# EUROPÄISCHE PATENTANMELDUNG

(11) **EP 4 039 294 A1**
(43) Veröffentlichungstag der Anmeldung: **10.08.2022**
(21) Anmeldenummer: 21155442.3
(22) Anmeldetag: 05.02.2021
(51) Int. Cl.: A61M 5/20, A61M 5/32

(54) **AUTOINJEKTOR**

(71) Anmelder: Muratov, Ruslan Ravilievich, Sestrorezk, Sankt Petersburg 197706 (RU)
(72) Erfinder: Muratov, Ruslan Ravilievich, Sestrorezk, Sankt Petersburg 197706 (RU)
(74) Vertreter: Jeck, Anton

(57) **Zusammenfassung**

Das Autoinjektionsgerät - Autoinjector - bezieht sich auf medizinische Geräte und kann im Gesundheitswesen verwendet werden, um Injektionen von flüssigen Medikamenten und anderen Flüssigkeiten für medizinische Zwecke durchzuführen.

Der Hauptbestandteil des Autoinjektors ist eine vorgefüllte Standardspritze vom Luer-Typ, die sich in einem zylindrischen Gehäuse aus einem für den medizinischen Gebrauch zugelassenen Polymermaterial befindet, in dem auch die Hauptbaugruppen des Geräts untergebracht sind:
eine Zuführungsbaugruppe, die das Starten der Vorrichtung, das Bewegen der Spritze zum Körper des Patienten, das Eintauchen der Nadel in den Körper senkrecht zur Hautoberfläche und das Durchführen der Injektion des flüssigen Medikaments in die Spritze sicherstellt;
eine Rückführungsbaugruppe, die das Entfernen der Nadel nach der Injektion und das Versetzen der Vorrichtung in einen nicht-operativen Zustand sicherstellt und die Wiederverwendung der Vorrichtung verhindert;
eine Verriegelungsbaugruppe, die die Spritze im Autoinjektorkörper während des Transports und der Lagerung der Vorrichtung fixiert und die spontane Betätigung der Vorrichtung verhindert.

Das vorgeschlagene Design des Autoinjektors zeichnet sich durch eine geringe Anzahl von Teilen, durch Einfachheit und durch Zuverlässigkeit aus und umfasst nur 11 Teile aus polymeren Materialien, die für den medizinischen Gebrauch zugelassen sind, und 2 Metallfedern.

## Beschreibung

Das automatische Injektionsgerät - Autoinjector - bezieht sich auf medizinische Geräte und kann im Gesundheitswesen für Injektionen von flüssigen Medikamenten und anderen Flüssigkeiten für medizinische Zwecke verwendet werden.

Spritzen als medizinisches Routinewerkzeug werden seit dem 19. Jahrhundert zum Ansaugen biologischer Flüssigkeiten, zur Verabreichung von Medikamentenlösungen und zum Anlegen von Infusionen verwendet. Ihre Entwicklung ist mit den Namen des Franzosen Charles-Gabriel Pravas und des Schotten Alexander Wood verbunden. In der Praxis haben sich verschiedene Spritzenvarianten bewährt. Die Pravas-Spritze bestand aus drei Elementen. Diese bestanden aus einem Glaszylinder mit Metallrahmen, einer Kanüle für eine Röhrennadel aus Silber oder Gold und einem graduierten Metallkolben aus Durit, Asbest oder vulkanisiertem Gummi. Woods Spritze bestand ebenfalls aus einer Hohlnadel und einem Zylinder, wurde aber nicht für chirurgische Zwecke, sondern für subkutane Injektionen verwendet.

Im Allgemeinen handelt es sich bei der Spritze um einen Hohlzylinder mit einer am Boden befestigten Nadel und einem hinten angeschlossenen Kolben, der aus einer Stange mit einer Dichtung am inneren Ende und einer Verlängerung am äußeren Ende zum Greifen besteht.

Alle Spritzen arbeiten nach dem gleichen Prinzip. Eine Nadel wird in ein Gefäß mit Flüssigkeit gesteckt. Dann wird der Kolben angehoben, wodurch eine Unterdruckzone unter dem Kolben entsteht. Die Flüssigkeit tritt unter dem Einfluss des atmosphärischen Drucks durch die Nadel in den geschlossenen Zylinder ein. Daraufhin wird die gefüllte Spritze mit der Nadel auf den Kopf gestellt. Durch leichtes Drücken des Kolbens werden Luftblasen, die in die Spritze eingedrungen sind, herausgedrückt. Die Injektion wird durchgeführt, indem die Nadel scharf in den Körper bis zur gewünschten Tiefe eingeführt wird. Der Spritzenzylinder wird mit zwei Fingern gehalten, und mit dem Daumen wird die Kolbenstange gedrückt. Dabei wird die Kraft so eingestellt, dass die Flüssigkeit aus der Spritze in den Körper des Patienten bewegt wird, wobei der Widerstand des biologischen Gewebes überwunden wird.

Solche medizinischen Manipulationen erfordern ein gewisses Maß an professioneller Ausbildung und sind mit schmerzhaften Empfindungen und Angst vor Hautstörungen für die Patienten verbunden. Das macht es schwierig, Injektionen in Notfällen selbständig durchzuführen.

Eine Alternative zu manuellen Spritzen für die Injektion von Substanzen in den Körper des Patienten sind automatische Injektionsgeräte, die es dem Patienten ermöglichen, sich selbst zu injizieren.

Automatisierte Injektionsgeräte wurden zur Verabreichung von Medikamenten unter Notfallbedingungen eingesetzt, z. B. zur Verabreichung von Ephedrin, um die Auswirkungen einer schweren allergischen Reaktion zu neutralisieren. Automatische Injektionsvorrichtungen wurden auch im Zusammenhang mit der Verabreichung von Antiarrhythmika und selektiven Thrombolytika während eines Herzinfarkts beschrieben (U.S. Patent Nr. 3,910,260; 4,004,577; 4,689,042; 4,755,169 und 4,795,433). Verschiedene Arten von automatischen Injektionsvorrichtungen sind beispielsweise auch in den US-Patentnummern 3.941.130; 4.261.358; 5.085.642; 5.092.843; 5.102.393; 5.267.963; 6.149.626; 6.270.479 und 6.371.939 sowie in der US-Patentveröffentlichung Nr. WO/2008/005315 beschrieben.

Eine herkömmliche automatische Injektionsvorrichtung zwingt bei Betätigung die im Gerät befindliche Spritze in eine Vorwärtsbewegung. Dabei ragt die Nadel aus ihrem Gehäusekäfig heraus, so dass die in der Spritze enthaltene Substanz in den Körper des Patienten injiziert wird. In einigen Fällen hilft es, die Spritze in Richtung der Haut des Patienten zu bewegen, so dass die Nadel in die Haut eindringt, bevor die Substanz aus dem inneren Bereich der Spritze injiziert wird. Dadurch wird verhindert, dass die Substanz aus der Nadel austritt, bevor die Injektion durchgeführt wird.

Es ist eine Vorrichtung zur Medikamenteninjektion bekannt (Patent RU 2431505, Prioritätsdatum 22.11.2005, publ. 20.10.2011, Coll. Nr. 29), die Folgendes enthält:
ein Gehäuse mit einem vorderen Ende, in dem sich ein Loch zum Durchführen einer Nadel befindet;
einen Hohlraum zum Aufnehmen eines Spritzenmoduls, der sich entlang der Länge des Gehäuses und angrenzend an das vordere Ende erstreckt und angepasst ist, um ein abnehmbares Spritzenmodul mit der Möglichkeit aufzunehmen, es mit Gleiten relativ zum vorderen Ende zu bewegen, und die Nadel des Spritzenmoduls durch das Loch zum Durchführen der Nadel nach außen gehen kann;
einen Spritzenschieber, der mit dem Gehäuse verbunden und mit einer Stange versehen ist, die in Richtung des vorderen Endes innerhalb des Hohlraums bewegt werden kann, um das Spritzenmodul aufzunehmen; und
eine Nadeleindringtiefensteuerung, die am vorderen Ende des Gehäuses angebracht und mit einer Halterung für das Spritzenmodul versehen ist, die in einem gewissen Abstand vom vorderen Ende in einer Position angeordnet ist, um die gewünschte Nadeleindringtiefe zu bestimmen.

Eine automatische Injektionsvorrichtung ist ebenfalls bekannt (Patent RU 2524487, Prioritätsdatum 29.04.2010, publ. 27.07.2014, Koll. Nr. 27) mit einer Spritze, die eine Spritzenhülse zur Aufnahme einer Substanz und einen Spritzenkolben aus einem polymeren Material aufweist. Dabei enthält der Spritzenkolben ein am proximalen Ende befindliches Druckelement und ein distales Ende, das in einen ersten Kolbenfuß mit einer ersten konischen Fläche und einer zweiten konischen Fläche gegabelt ist, und einen zweiten Kolbenfuß mit einer ersten konischen Oberfläche und einer zweiten konischen Oberfläche. Das distale Ende weist eine erste Kontaktfläche auf, die durch die erste konische Oberfläche des ersten Kolbenfußes und die erste konische Oberfläche des zweiten Kolbenfußes definiert ist. Dabei ist die erste Kontaktfläche so ausgebildet, dass sie in anfänglichem Kontakt mit dem Auslösemechanismus steht. Die erste Kontaktfläche ist in einem ersten Winkel zwischen etwa 40° und etwa 80° relativ zur Längsachse des Spritzenkolbens angeordnet. Eine zweite Kontaktfläche ist durch eine zweite konische Oberfläche des ersten Kolbenfußes und eine zweite konische Oberfläche des zweiten Kolbenfußes definiert. Dabei ist die zweite Kontaktfläche so ausgebildet, dass sie nach dem Kontakt mit dem Auslösemechanismus verbunden ist. Das technische Ergebnis ist, dass die Möglichkeit einer unbeabsichtigten Bewegung des Stößels ausgeschlossen ist.

Das nächstliegende Analogon (Prototyp) ist ein Autoinjektor (Patent RU 2585693, Prioritätsdatum 06.10.2011, veröffentlicht 10.06.2016, Slg. Nr. 16), bei dem es sich um ein röhrenförmiges Gestell handelt. Dieses enthält eine Spritze mit einer Hohlnadel, einen Stopfen zum Verschließen der Spritze und zum Bewegen des Medikaments, eine Antriebsfeder zum Bereitstellen einer Bewegung der Spritze zur Abgabe einer Medikamentendosis, eine Einstellfeder, die die Nadel aus einer geschlossenen Position innerhalb des Gestells in eine ausgefahrene Position durch eine Öffnung zum Einführen der Nadel schieben kann und das Gerüst nach der Injektion über die Nadel vorschieben kann, und ein Betätigungsmittel, das die Betätigungs- und Einstellfedern bis zur manuellen Betätigung in einem zusammengedrückten Zustand arretiert und bei manueller Betätigung die Einstell- und/oder Betätigungsfedern zur Injektion freigibt.

Die Merkmale der oben genannten Geräte sind ihre erhöhte Komplexität und das Vorhandensein einer großen Anzahl von Teilen aus verschiedenen Materialien. Die Folge davon sind hohe Kosten.

Ziel der Erfindung ist es, die Konstruktion unter Beibehaltung der notwendigen Funktionalität zu vereinfachen, d. h. die sichere Platzierung des vorgefüllten Spritzenmoduls im Autoinjektorkörper unter Beibehaltung seiner Sterilität zu gewährleisten sowie die notwendigen manuellen Handgriffe bei der Vorbereitung der Vorrichtung für den Gebrauch und ihre Inbetriebnahme so weit wie möglich zu vereinfachen.

### WESEN DER ERFINDUNG

Der Autoinjektor-Muratov ist eine Vorrichtung zur automatischen Injektion von flüssigen Medikamenten, deren Hauptbestandteil eine vorgefüllte Standardspritze vom Luer-Typ ist, die sich in einem zylindrischen Gehäuse aus einem für den medizinischen Gebrauch zugelassenen Polymermaterial befindet, in dem auch die Hauptknoten der Vorrichtung untergebracht sind, nämlich:
- eine Zuführungseinheit, die das Gerät startet, die Spritze zum Körper des Patienten bewegt, die Nadel senkrecht zur Hautoberfläche in den Körper eintaucht und das in der Spritze enthaltene flüssige Medikament injiziert;
- eine Rückholvorrichtung, die sicherstellt, dass die Nadel nach der Injektion entfernt und das Gerät funktionsunfähig gemacht wird, um eine Wiederverwendung des Geräts zu verhindern;
- eine Verriegelungsbaugruppe, die die Spritze während des Transports und der Lagerung des Geräts im Körper des Autoinjektors verriegelt und eine spontane Auslösung des Geräts verhindert.

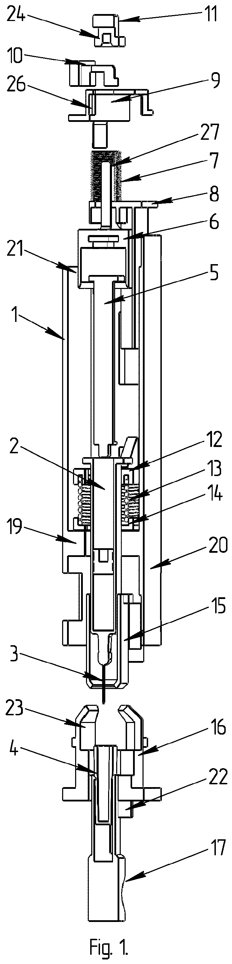

Der allgemeine Aufbau des Muratov-Autoinjektors ist in Fig. 1 - 6 dargestellt, wobei die einzelnen Teile, Einheiten und Fragmente des Geräts mit Nummern gemäß der folgenden Tabelle der Referenzpositionen bezeichnet sind.

**Tabelle 1. Referenzpositionen**

| Nº | Name des Teils, der Baugruppe oder des Fragments |
|---|---|
| 1 | Auto-Injektor -Gehäuse |
| 2 | Spritzenzylinder |
| 3 | Spritzen-Nadel |
| 4 | Spritzenschutzkappe |
| 5 | Spritzenschaft |
| 6 | Spindelschieber |
| 7 | Feder der Vorschubeinheit |
| 8 | Anschlag der Zuführeinheit |
| 9 | Tastenhalter |
| 10 | Stopper |
| 11 | Auslösetaste |
| 12 | Anschlag der Rücklaufgarnitur |
| 13 | Federrücklaufeinheit |
| 14 | Halterung für Rücklaufgarnitur |
| 15 | Führungsbuchse der Umlenkeinheit |
| 16 | Abschließbarer Deckel |
| 17 | Abnehmbarer Schaft |
| 18 | Spritzenkolbendichtung |
| 19 | O-Ring-Lasche zum Halten der Rücklaufeinheit |
| 20 | Abschließbarer Deckelbefestigungsbereich |
| 21 | Anbauraum der Zuführeinheit |
| 22 | Schafthalterung |
| 23 | Verriegelungslasche der Verriegelungsabdeckung |
| 24 | Schlitz im Taster für den Stopper |
| 25 | Trittbrett zum Einschieben des Stopfens |
| 26 | Verriegelungslasche des Tastenhalters |
| 27 | Vorschubfederhalter |
| 28 | Tiefe zum Verriegeln des Verschlussdeckels |
| 29 | Tiefe für die Befestigung des Tastenhalters |
| 30 | Schlitz im Gehäuse für den Stopfen |
| 31 | Ausziehbares Gerätehalter-Blatt |
| 32 | Schlitz zur Befestigung der Klinge des Umlenkrollenhalters |

Fig. 1 zeigt eine Gesamtansicht des Gerätes im Schnitt, wobei die einzelnen Teile und Baugruppen im Folgenden beschrieben werden.

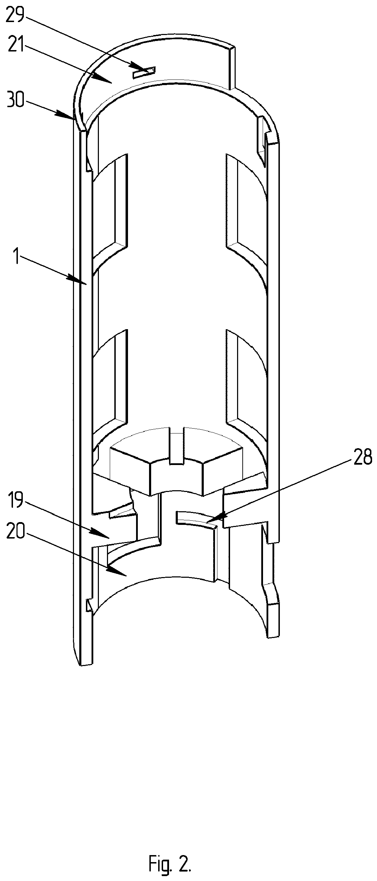

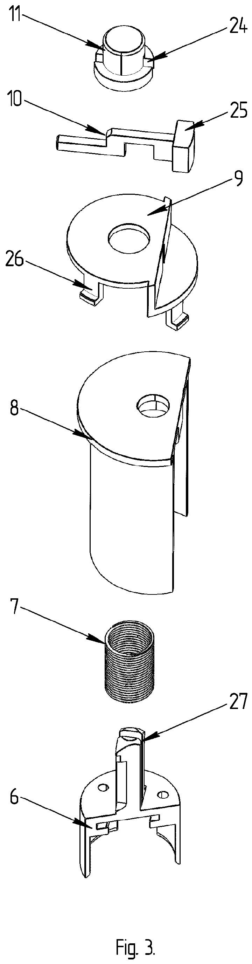

Der Aufbau des Gehäuses 1 ist in Fig. 2 dargestellt. Im oberen Teil des zylindrischen Körpers befindet sich eine Zone 21 zur Befestigung der Vorschubeinheit (Fig. 3) in Form einer Ringablage zur Befestigung des Anschlags 8 und des Halters 9 der Starttaste/ Auslösetaste 11, die bei der Montage der Vorrichtung in den Nuten 29 des Auto-Injektor-Gehäuse 1 mit Hilfe von Befestigungslaschen 26 starr befestigt sind, sowie ein Schlitz 30 für einen Stopper.

Im unteren Viertel des Gehäuses 1 befindet sich ein ringförmiger Vorsprung 19, der den mittleren Bereich des Gehäuses, in dem das Spritzenmodul (Fig. 4) und die Rückführeinheit (Fig. 5) untergebracht sind, vom Endbereich 20 trennt, in dem sich eine Verriegelungseinheit (Fig. 6) befindet.

Der Aufbau der Baugruppenteile des Feeders ist in Fig. 3 dargestellt. Der Spindelschieber 6 ist das Hauptelement der Vorschubeinheit, das sich im oberen Teil des Gehäuses befindet und an seinem oberen Teil einen Halter 27 hat, der aus zwei parallelen Blütenblättern besteht. Auf denen ist die Feder 7der Vorschubeinheit, die durch einen Anschlag 8 in zusammengedrücktem Zustand gehalten wird, durch Vorsprünge an den Enden der Blütenblätter befestigt. Zwischen diesen ist ein Stopper 10 angebracht, der den Startknopf/ Auslösetaste 11 in der Ausgangsposition fixiert und verhindert, dass sich die Blütenblätter vor der Benutzung der Vorrichtung zusammenfinden.

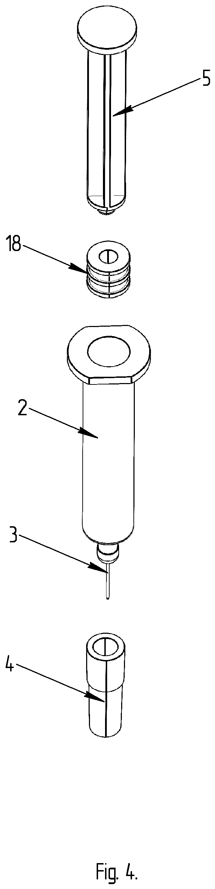

Der zentrale Teil des Gehäuses 1 nimmt eine vorgefüllte modulare Standardspritze vom Luer-Typ auf, deren Aufbau in Fig. 4 dargestellt ist. Sie besteht aus einem zylindrischen Glaskörper 2, in dessen Ende eine hohle Injektionsnadel 3 eingeschmolzen ist, die mit einer sterilen Schutzkappe 4 verschlossen ist, einem Spritzenschaft 5 mit einer Dichtung 18 an seinem inneren Ende und einem flachen Anschlag am gegenüberliegenden Ende. Dieser ist in den Schlitz des Spindelschieber 6 eingesetzt. Der Spritzenkörper wird in die zylindrische Führungshülse 15 gesteckt, die Teil der Rücklaufeinrichtung ist und unten eine Verjüngung aufweist, die als Anschlag für den Spritzenkörper dient.

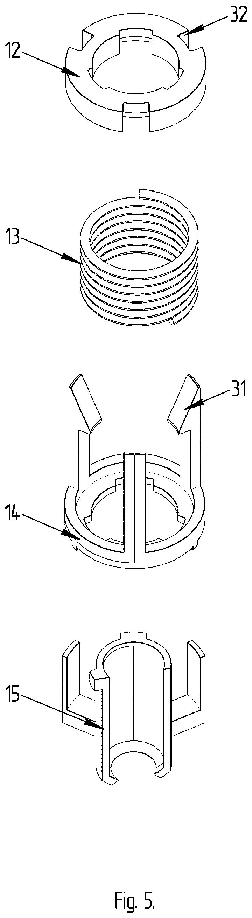

Der Aufbau der Teile der Rücklaufgarnitur ist in Fig. 5 dargestellt. Die Basis der Rückholvorrichtung ist der Halter 14, der aus einem flachen Ring besteht, von dem sich drei biegsame/ausziehbare Gerätehalter-Blätter 31 senkrecht erstrecken, mit einer Rückholfeder/ Federrücklaufeinheit 13, die durch einen Anschlag 12 der Rücklaufgarnitur mit Schlitzen zur Befestigung der Vorsprünge auf den Gerätehalter-Blätter 31 zusammengedrückt wird. Der Innendurchmesser des Halters 14 entspricht dem Außendurchmesser der Führungshülse 15, so dass sich diese koaxial und ohne Reibung im Innenvolumen der Rückführeinheit bewegen kann, während sie den Spritzenzylinder führt. Dabei liegt der Halter 14 auf dem ringförmigen Vorsprung 19 des Auto-Injektor-Gehäuse 1 an dessen Boden auf.

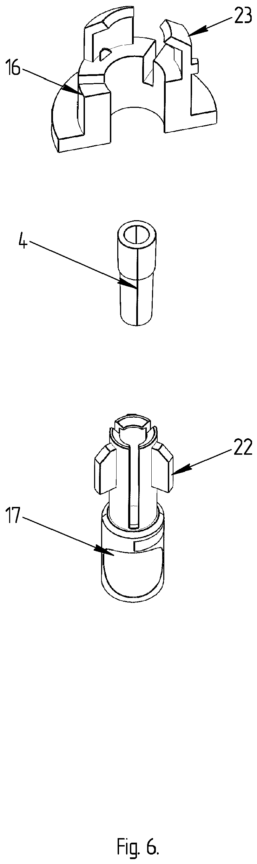

Im unteren Teil des Gehäuses befindet sich eine Verriegelungsbaugruppe, deren Aufbau in Fig. 6 dargestellt ist. Der Hauptteil der Verriegelungsbaugruppe ist eine Verriegelungskappe 16, die fest in den unteren Teil des Gehäuses 1 eingesetzt und mittels dreier Vorsprünge 23 in Aussparungen 28 im Inneren des Gehäuses 1 befestigt ist, mit einem abnehmbaren Schaft 17, der in Schlitze der Verriegelungskappe 16 eingesetzt ist und die Schutzkappe 4 der Spritze fest abdeckt. Die Verriegelungsbaugruppe sichert die Spritze im Autoinjektorkörper während des Transports und der Lagerung des Geräts und verhindert ein spontanes Auslösen des Geräts.

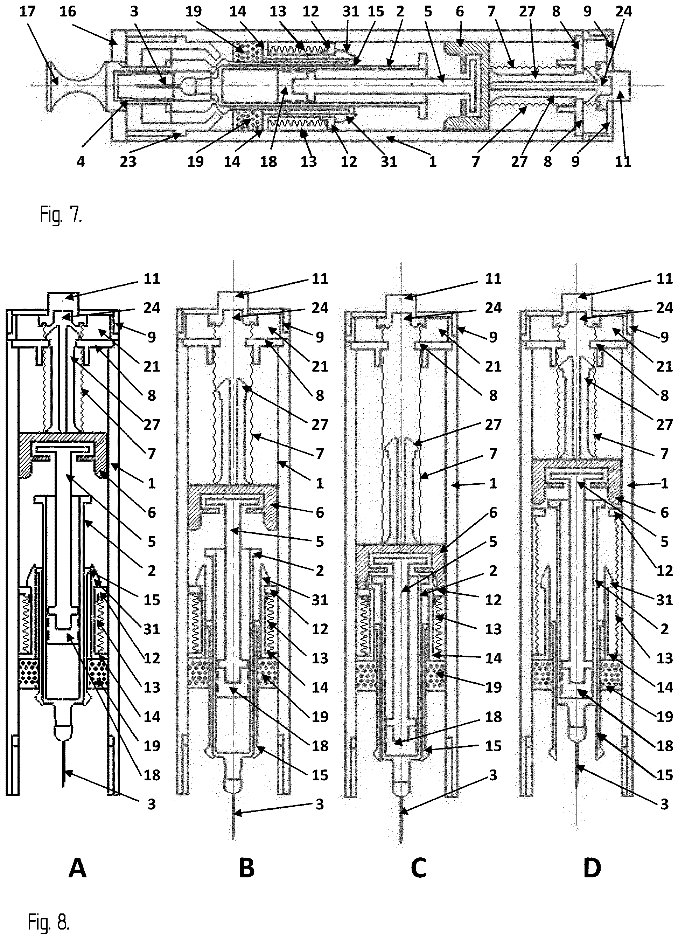

Die Funktionsweise des Geräts wird durch die Abbildungen in Fig. 7 und 8 verdeutlicht, die das Zusammenfügen von Teilen und Baugruppen in fünf verschiedenen Phasen zeigen, nämlich:
- Phase 0 - Anfangszustand der Vorrichtung während der Lagerung oder des Transports (Fig. 7), in dem die vorgefüllte Spritze im sterilen Zustand von unten durch die Verschlusskappe 16 und von oben durch den Stopper 10 blockiert ist. Beide Federn 7 und 13 sind im zusammengedrückten Zustand;
- Phase A - betriebsbereiter Zustand, zu dessen Eintritt der Bediener durch Drehen des Schaftes 17 die Verschlusskappe 16 löst und sie zusammen mit der Schutzkappe 4 der Spritze vom Körper entfernt;
- Phase B - der Zustand des Einführens der Nadel in den Körper des Patienten, zu dessen Eintritt der Bediener den Körper des Autoinjektors mit vier Fingern seiner Hand ergreift, sein unteres Ende gegen den Körper des Patienten drückt, mit dem Zeigefinger den Stopper 10 bewegt und mit dem Daumen die Starttaste/ Auslösetaste 11 drückt, Infolgedessen bringt der Knopf/ Auslösetaste 11 die Blütenblätter 27 zusammen, und die Feder 7 setzt die Schubstange 6 in Bewegung. Die Spritze 2 bewegt sich zusammen mit der Führungshülse 15 entlang des Auto-Injektor-Gehäuse 1 und taucht die Nadel 3 in den Körper des Patienten ein, während die Vorsprünge an der Oberseite der Führungshülse 15 die Bewegung der Spritze 2 auf die Länge der Nadel 3 begrenzen;
- Die Phase C - Injektion - folgt unmittelbar auf die Phase B als deren Fortsetzung, während der Spritzenzylinder 2 stationär bleibt und die von der Feder von flüssigen Medikamenten von etwa 100 µl/s;
- Phase D - Rückzug der Nadel - tritt auf, wenn die Schubstange 6 nach Beendigung der Injektion auf die Gerätehalter-Blätter 31 der Rückholhalterung 14 drückt und den Anschlag 12 entriegelt, wodurch die Feder 13 freigegeben wird. Deren Kraft wird auf den ringförmigen Vorsprung des Spritzenzylinders 2 in entgegengesetzter Richtung zur Kraft der Vorschubfeder 7 aufgebracht und bewegt die Spritze in ihre Ausgangsposition, wobei die Nadel 3 vollständig aus dem Körper des Patienten herausgezogen wird.

Die vorgeschlagene Konstruktion des Autoinjektors zeichnet sich durch eine geringe Anzahl von Teilen, durch Einfachheit und durch Zuverlässigkeit aus, enthält nur 11 Teile aus polymeren Materialien, die für den medizinischen Gebrauch zugelassen sind, und zwei Metallfedern. Standardmodule - vorgefüllte Spritzen - werden in Massenchargen produziert, sind preiswert und verfügbar.

## Patentansprüche

1. Vorrichtung zur automatischen Injektion eines flüssigen Medikaments, umfassend einen zylindrischen Körper, der eine vorgefüllte Standardspritze enthält, eine Zuführungsbaugruppe, die zum Starten der Vorrichtung, zum Bewegen der Spritze zum Körper des Patienten, zum Eintauchen der Nadel in den Körper des Patienten und zum Injizieren des flüssigen Medikaments in der Spritze vorgesehen ist, eine Rückführungsbaugruppe, die zum Entfernen der Nadel nach der Injektion und zum Versetzen der Vorrichtung in einen nicht betriebsbereiten Zustand, der eine Wiederverwendung der Vorrichtung verhindert, vorgesehen ist, eine Verriegelungsbaugruppe zur Fixierung der Spritze im Autoinjektorkörper während des Transports und der Lagerung der Vorrichtung und Verhinderung einer spontanen Aktivierung der Vorrichtung,
**dadurch gekennzeichnet,**
**dass** der obere Teil des zylindrischen Körpers (1) eine Ringablage zur Fixierung des Anschlags (8) der Zuführeinheit und der Halterung (9) des Startknopfes (11) aufweist, starr befestigt bei der Montage der Vorrichtung in Nuten (29) des Gehäuses (1) mittels Befestigungslaschen (26) sowie einem Schlitz (30) für einen Stopfen, und im unteren Viertel des Gehäuses die ringförmige Schulter, auf der die Halterung der Rücklaufeinheit aufliegt.

2. Vorrichtung nach Anspruch 1
**dadurch gekennzeichnet,**
**dass** im mittleren Teil des Gehäuses (1) die standardmäßig vorgefüllte modulare Spritze mit einem flachen Anschlag am gegenüberliegenden Ende eines Stabes in der Nut der Schubstange (6) angeordnet ist.

3. Vorrichtung nach Anspruch 1 oder 2
**dadurch gekennzeichnet,**
**dass** die Schubstange (6) an ihrem oberen Teil einen Halter (27) aufweist, der aus zwei parallelen Lappen besteht, auf denen eine Feder (7) montiert ist und durch einen Anschlag (8) zusammengedrückt gehalten wird, der durch Laschen an den Enden der Lappen befestigt ist, zwischen denen sich ein Stopper (10) befindet (11), der den Abzug (11) in der Startposition verriegelt und verhindert, dass die Klingen vor der Verwendung zusammenkommen.

4. Vorrichtung nach einem der Vorherigen Ansprüche
**dadurch gekennzeichnet,**
**dass** der Spritzenzylinder in eine zylindrische Führungshülse (15) eingesetzt wird, die am Boden eine Verjüngung aufweist, die als Anschlag für den Spritzenzylinder dient,
**dass** der Spritzenzylinder (15) eine Rückstoßfeder (13) aufweist, die innerhalb des Halters (14) enthalten ist, der aus einem flachen Ring mit drei Gerätehalter-Blätter (31) besteht, die senkrecht zum Ring angeordnet sind und im zusammengedrückten Zustand durch den Anschlag (12) gehalten werden, der geschlitzt ist, um in die Gerätehalter-Blätter (31) einzugreifen.

5. Vorrichtung nach einem der Vorherigen Ansprüche
**dadurch gekennzeichnet,**
**dass** der untere Teil des Trägers (14) von einem kreisförmigen Vorsprung (19) des Gehäuses (1) getragen wird.

6. Vorrichtung nach einem der Vorherigen Ansprüche
**dadurch gekennzeichnet,**
**dass** der Verriegelungsdeckel (16), der sich im unteren Teil des Gehäuses befindet, durch drei Laschen (23) in Aussparungen (28) im Inneren des Gehäuses (1) gesichert wird, wobei ein abnehmbarer Schaft (17) in die Schlitze des Verriegelungsdeckels (16) eingesetzt wird.
